# EUROPÄISCHE PATENTSCHRIFT

(11) **EP 3 821 919 B1**
(45) Veröffentlichungstag und Bekanntmachung des Hinweises auf die Patenterteilung: **07.06.2023**
(21) Anmeldenummer: 21150379.2
(22) Anmeldetag: 07.07.2016
(51) Int. Cl.: A61L 27/22, A61L 31/04

(54) **VERFAHREN ZUR HERSTELLUNG EINES BIOARTIFIZIELLEN, PRIMÄR AZELLULÄREN KONSTRUKTS AUF FIBRINBASIS UND DIESES KONSTRUKT SELBST**
METHOD FOR PRODUCING A BIOARTIFICIAL PRIMARILY ACELLULAR FIBRIN-BASED CONSTRUCT AND THE CONSTRUCT ITSELF
PROCÉDÉ DE FABRICATION D'UNE CONSTRUCTION ACELLULAIRE PRIMAIRE BIOARTIFICIELLE À BASE DE FIBRE ET CONSTRUCTION

(30) Priorität: 09.07.2015 DE 102015111126
(43) Veröffentlichungstag der Anmeldung: 19.05.2021
(62) Teilanmeldung aus: 16736169.0
(73) Patentinhaber: Medizinische Hochschule Hannover, 30625 Hannover (DE)
(72) Erfinder: APER, Thomas, 30629 Hannover (DE); WILHELMI, Mathias, 30916 Isernhagen (DE); HAVERICH, Axel, 30177 Hannover (DE)
(74) Vertreter: Kröncke, Rolf

(56) Entgegenhaltungen:
- WO-A1-2013/091865
- WO-A2-03/035115
- DE-T2- 60 222 543
- US-A- 3 723 244
- THOMAS APER: "Fibrinogen Preparations for Tissue Engineering Approaches", JOURNAL OF BIOENGINEERING AND BIOMEDICAL SCIENCES, Bd. 02, Nr. 03, 1. Januar 2012 (2012-01-01), XP055303757, DOI: 10.4172/2155-9538.1000115

## Beschreibung

Die vorliegende Erfindung richtet sich auf ein Verfahren zur Herstellung eines bioartifiziellen und primär azellulären Konstrukts auf Fibrinbasis, wobei eine Mischung aus Fibrinogen-haltigen und Thrombin-haltigen zellfreien Zusammensetzungen auf einer Oberfläche aufgebracht und zumindest solange druckbeaufschlagt wird, bis die Fibrinbildung im Wesentlichen abgeschlossen ist, wobei die Beaufschlagung flächig auf die auf der Oberfläche aufgebrachten Zusammensetzungen erfolgt, wobei die Druckbeaufschlagung mit einem relativen Druck von mindestens 100 kPa (1 bar) erfolgt und wobei die aufgebrachte Schicht eine Dicke von mindestens 1 mm aufweist. In einem weiteren Aspekt richtet sich die vorliegende Erfindung auf solche erfindungsgemäß erhaltenden bioartifiziellen, azellulären Konstrukte auf Fibrinbasis mit verbesserten biomechanischen Eigenschaften, sowie die Konstrukte zur Verwendung in Verfahren im Bereich der Implantologie, Knorpelersatz oder Gewebeersatz.

### Stand der Technik

Das Gebiet des Tissue Engineerings und hier insbesondere der Einsatz von Tissue Engineering für medizinische Transplantationstechnik nimmt eine immer größere Stellung bei der Versorgung von Patienten ein. Hier spielt die Herstellung von artifiziellen, biologischen (Gewebe)konstrukten, die in der medizinischen Transplantationstechnik eingesetzt werden können, eine immer wichtigere Rolle.

Seit mehr als 30 Jahren wird auf dem Gebiet des Tissue Engineerings daran gearbeitet, körpereigene Materialien, wie Organe, Gefäße oder auch Gewebepatches möglichst gleichwertig durch im Labor hergestellte artifizielle Gewebekonstrukte aus biologischen und gegebenenfalls körpereigenen Materialien zu ersetzen, die als medizinische Transplantate verwendet werden können. Die häufigsten Ansatzpunkte sind dabei solche, bei denen Gewebekonstrukte bereitgestellt werden, die *in vitro* derart generiert werden, dass diese als funktionsfähige Implantate üblicherweise mit Zellen in den Körper eingebracht werden. Das heißt, dass die Gewebeimplantate bereits vollständig mit entsprechenden Zellen besiedelt und ausdifferenziert sind, so dass die Generierung des Gewebes vollständig *in vitro* erfolgt und anschließend dieses Gewebekonstrukt implantiert wird. Es besteht ein hoher Bedarf geeignete bioartifizielle Konstrukte bereitzustellen, die verschiedenste Erfordernisse erfüllen müssen. Sie dürfen im Körper selbst keine unerwünschten Reaktionen hervorrufen, wie Abstoßungsreaktionen usw. Das heißt, die Bio- und ggf. Hämokompatibilität der Konstrukte steht im Vordergrund. Das artifizielle Konstrukt soll nach Möglichkeit im Körper so angenommen werden, dass es nicht als fremd erkannt wird und keine pathologischen Reaktionen ausgelöst werden.

Darüber hinaus ist zu differenzieren, ob das Konstrukt eines mit einer Matrix ist, das permanent im Körper verbleiben soll oder eines mit einer Matrix, das sich über die Zeit im Körper auflöst.

Hierbei wird unter Gewebekonstrukten üblicherweise ein Erzeugnis verstanden, dass aus einem Matrixmaterial und aus differenzierten und/oder undifferenzierten Zellen gebildet wird, wobei diese Bildung *in vitro* stattfindet. Derzeit wird versucht, solche bioartifiziellen Gewebekonstrukte als Ersatz für synthetische Materialien zu verwenden. Als Matrixmaterial wird hierbei ein natürliches, biologisches Material eingesetzt, zum Beispiel extrazelluläre Matrizes wie sie in der Natur vorkommen. Solche Materialien sind gewünscht, um entsprechend im Körper tolerierbar zu sein.

Alternativ können permanente Strukturen in das Gewebekonstrukt als Matrizes eingesetzt werden, wie synthetische Strukturen einschließlich Kunststoffstrukturen oder Metallstrukturen. Diese Strukturen verbleiben permanent im Körper oder müssen gegebenenfalls durch einen zweiten operativen Eingriff entfernt werden.

Aus dem Stand der Technik sind verschiedenste Verfahren zur Generierung von bioartifiziellen Gewebekonstrukten bekannt. Beispielhaft wird hier auf die WO 2013/091865 A1 verwiesen, das ein Verfahren und eine Vorrichtung zur Herstellung von bioartifiziellen Gewebekonstrukten mithilfe von Rotationsverfahren beschreibt. Dabei werden bioartifizielle Gewebekonstrukte hergestellt, mit denen Zellen-Matrix-Verbände erzeugt werden. Hierbei werden die Zellen direkt zur *in situ*-Generierung des Gewebekonstrukts eingesetzt. Die meisten Verfahren zur Herstellung von entsprechenden Gewebekonstrukten beinhalten, dass *in vitro* die Zellen in oder auf die Matrix aufgebracht und gegebenenfalls anschließend kultiviert werden, um zuerst ein Gewebekonstrukt auszubilden. Dieses wird dann in den Körper implantiert.

Ein Problem solcher Gewebekonstrukte ist, dass diese meist in Einzelanfertigung zur direkten Anwendung erzeugt werden. Das heißt, dem Patienten werden in einem ersten Schritt Zellen entnommen, die dann über einen gewissen Zeitraum in oder auf der Matrix kultiviert werden. Nach *in vitro* Generierung des Gewebekonstrukts wird dieses in den Körper des Empfängers implantiert. Solche Verfahren erfordern umfangreiche Schritte und aufwendige Einrichtungen. Weiterhin erfordern solche Verfahren und die Generierung von solchen Konstrukten umfangreiche Zulassungsverfahren, die einen Einsatz hiervon erschweren. Daher besteht ein Bedarf neue Verfahren bereitzustellen, die die Generierung von bioartifiziellen Konstrukten erlaubt, die schnell sicher und einfach hergestellt werden können, die eine hervorragende Lagerstabilität aufweisen und deren Einsatz keine umfangreichen singulären Zulassungserfordernisse benötigen.

US 3723244 beschreibt die Herstellung von Fibrin-Konstrukten unter Kompaktieren von zellfreien Fibrinmonomer-haltigen Suspensionen/Lösungen durch Ausübung von Zentrifugalkräften.

Weiterhin ist es Ziel der vorliegenden Erfindung Konstrukte bereitzustellen, die auch komplexere Strukturen nachbilden können. Die in der WO 2013/091865 A1 beschriebenen Strukturen sind Hohlkörper, die zwar als entsprechende Hohlkörper eingesetzt werden können, für die Ausbildung von anderen Strukturen ist es aber notwendig, dass der Hohlkörper aufgeschnitten und in entsprechende gewünschte Strukturen umgeformt wird. Dabei werden diese dort hergestellten Gewebekonstrukte allerdings neuen Kräften ausgesetzt, die die mechanischen Eigenschaften dieser Gewebekonstrukte verschlechtern können.

Entsprechend ist eine weitere Aufgabe der vorliegenden Erfindung die Bereitstellung von Konstrukten mit verbesserten mechanischen Eigenschaften.

Zur Lösung dieser Aufgaben werden mit einem Verfahren nach Anspruch 1 und entsprechende Konstrukte nach Ansprüchen 12 bzw. 15 sowie deren Verwendung nach Anspruch 14 bereitgestellt.

### Beschreibung der Erfindung

Das erfindungsgemäße Verfahren ist ein Verfahren zur Herstellung eines bioartifiziellen und azellulären Konstrukts auf Fibrinbasis mit den Schritten:
- Bereitstellung einer Fibrinogen-haltigen und zellfreien Zusammensetzung;
- Bereitstellung einer Thrombin-haltigen und zellfreien Zusammensetzung;
- Aufbringen der Fibrinogen-haltigen zellfreien und der Thrombin-haltigen zellfreien Zusammensetzungen auf eine Oberfläche;
- Druckbeaufschlagung der auf der Oberfläche aufgebrachten Mischung der Zusammensetzungen zumindest solange, bis die Fibrinbildung im Wesentlichen abgeschlossen ist;
- Entfernen des azellulären bioartifiziellen Konstrukts auf Fibrinbasis von der Oberfläche, wobei die Beaufschlagung flächig auf die Oberfläche aufgebrachten Zusammensetzungen erfolgt, wobei die Druckbeaufschlagung mit einem relativen Druck von mindestens 100 kPa [1 bar], wie mindestens 500 kPa [5 bar] erfolgt und wobei die aufgebrachte Schicht eine Dicke von mindestens 1 mm aufweist.

Erfindungsgemäß wird dabei unter einem "bioartifiziellen und azellulären Konstrukts auf Fibrinbasis" ein Konstrukt verstanden, in dem primär keine Zellen vorliegen. D.h. währende des Herstellungsverfahrens werden keine Zellen in das Konstrukt eingebracht. Dieses Konstrukt ist auf Fibrinbasis, das heißt die Matrix dieses Konstrukts ist im Wesentlichen eine Fibrinmatrix oder weist eine Schicht auf, die im Wesentlichen eine Fibrinmatrix ist.

Unter dem Ausdruck "Matrix" wird eine Stützstruktur verstanden, die durch Fibrin ausgebildet wird. Die Matrix ist dabei eine provisorische Matrix (das heißt, sie löst sich nach Implantation auf), die ein Stützgerüst ausbildet und dem Konstrukt die entsprechende mechanische Festigkeit verleiht.

Das Konstrukt kann neben der aus Fibrin bestehenden Matrix weitere Schichten oder andere nicht zelluläre Bestandteile aufweisen.

Vorliegend werden die Ausdrücke "zellfrei" und "azellulär" synonym verwendet.

Das Verfahren beinhaltet, dass eine Fibrinogen-haltige und zellfreie Zusammensetzung bereitgestellt wird, eine Thrombin-haltige und zellfreie Zusammensetzung bereitgestellt wird und diese Zusammensetzungen auf eine Oberfläche eines Substrats aufgebracht werden.

In einer Ausführungsform wird die Fibrinogen-haltige Zusammensetzung aus Plasma mittels herkömmlicher Verfahren gewonnen. Solche Verfahren schließen Kryoprecipitation oder Ethanolprecipitation ein. Dabei kann es sich bei dem Plasma um eines handeln, dass von dem Empfänger des zukünftigen, erfindungsgemäßen Konstrukts stammt oder um allogenes oder xenogenes Fibrinogen bzw. Plasma. Gegebenenfalls kann die Fibrinogen-haltige Zusammensetzung vorab Wasch- und/oder Aufreinigungsschritten unterworfen werden, um zum Beispiel andere Bestandteile, wie Fette oder Albumine, abzutrennen. Die Zusammensetzung kann auch synthetisches oder rekombinantes Fibrinogen aufweisen.

In einer Ausführungsform wird dabei das separierte Fibrinogen aus dem Plasma derart resuspendiert, dass die Konzentration des Fibrinogens maximal 60 mg/ml wie maximal 50 mg/ml beträgt, wie maximal 20 mg/ml. In einer Ausführungsform wird die Konzentration des Fibrinogens in der Fibrinogen-haltigen Zusammensetzung derart eingestellt, dass diese eine Konzentration im Bereich von 10 bis maximal 20 mg/ml aufweist. Die Resuspension kann z.B. mit dem Plasma erfolgen. Zum Beispiel werden aus 50 ml Plasma 8 bis 10 ml Fibrinpräparation erhalten.

Das Fibrinogen liegt dabei in einer Ausführungsform in einer relativ stark verdünnten Konzentration vor, die Lösung ist sehr wässrig und verteilt sich sehr gut auf dem Substrat. Durch die geringe Konzentration findet die Vernetzung des Fibrinogens langsam statt.

Die Thrombin-haltige Zusammensetzung ist in einer Ausführungsform eine Thrombin-haltige Lösung, die Thrombin in einer Menge von 100-1000 Einheiten pro ml aufweist. Als Lösungsmittel kann zum Beispiel Aqua ad iniectabilia eingesetzt werden. Dem Fachmann sind sowohl für die Fibrinogen-haltige Zusammensetzung als auch für die Thrombin-haltige Zusammensetzung geeignete Lösungsmittel bekannt.

In einer Ausführungsform wird bei Verwendung einer relativ stark verdünnten Fibrinogenlösung, wie einer Fibrinogen-haltigen Lösung mit einer Fibrinogenkonzentration von maximal 20 mg/ml, eine Thrombinlösung vorab zugefügt, so dass das Verhältnis von Thrombin (Einheiten pro ml) zu Fibrinogen (mg pro ml) in einem Bereich von 1:1 bis 10:1 liegt.

Nach Aufbringen der Fibrinogen-haltigen azellulären und der Thrombin-haltigen azellulären Zusammensetzung auf einer Oberfläche wird eine Druckbeaufschlagung der auf der Oberfläche aufgebrachten Mischung der Zusammensetzungen durchgeführt. Diese Druckbeaufschlagung, die unmittelbar nach Aufbringen der Zusammensetzungen erfolgen kann, aber auch derart, dass die Druckbeaufschlagung erst nach einem vorbestimmten Zeitpunkt erfolgt, erfolgt zumindest solange, bis die Fibrinbildung im Wesentlichen abgeschlossen ist. Dieses sollte in einem Zeitraum von maximal 30 bis 45 min abgeschlossen sein und hängt vom Mischungsverhältnis und Konzentration der Reaktanten, Fibrinogen und Thrombin, ab.

Das heißt, die Druckbeaufschlagung erfolgt derart, dass die Polymerisation des Fibrinogens durch das Thrombin zur Ausbildung des Fibrinogens zumindest teilweise unter Druck erfolgt. Fibrin ist das polymerisierte Fibrinogen, das mithilfe des Thrombins vernetzt wird. Es bilden sich Fibrillen aus, die zu Fibrinfasern führen. Dabei bildet sich ein dreidimensionales Gebilde, eine Fibrinmatrix aus.

Es wird davon ausgegangen, dass aufgrund der Druckbeaufschlagung die Fibrinogenstrukturen näher zueinander gebracht werden, so dass eine verbesserte und verstärkte Vernetzung des Fibrinogens zum Fibrin erfolgen kann. Dadurch können verbesserte mechanische Eigenschaften dieser Fibrinstruktur erreicht werden. Die Fibrillen des Fibrinogens werden in einen engeren Kontakt gebracht, eine stärkere Quervernetzung zwischen den Fibrillen erfolgt. Diese so im Vergleich zu einer nicht-druckbeaufschlagten Fibrinmatrix verdichtete Fibrinmatrix weist eine höhere Stabilität gegenüber Scherung und Druckbeaufschlagung auf. Die Gefahr von Schäden dieser Struktur in einem Konstrukt, das in einen Körper eingebracht wird, ist verringert. Ein Problem der derzeitig hergestellten Gewebekonstrukte, insbesondere solche die starken mechanischen Belastungen ausgesetzt sind, wie Gefäßprothesen oder Knorpel, weisen das Problem einer geringen mechanischen Stabilität für diese Verwendung auf. Entsprechend wird im Stand der Technik versucht, solche Gewebekonstrukte durch *in vitro* Kultivierung mit entsprechenden Zellen zu stabilisieren um diese dann als Gewebekonstrukte zu implantieren.

Es zeigte sich nun, dass durch entsprechende Druckbeaufschlagung Fibrinmatrizes erreicht werden, die als bioartifizielle und azelluläre Konstrukte auf Fibrinbasis für Implantate verwendet werden können.

Neben den verbesserten mechanischen Eigenschaften weisen diese Konstrukte auch eine verbesserte Lagerungsfähigkeit auf. Dadurch, dass diese Konstrukte keinerlei Zellen enthalten, kann eine hervorragende Lagerungsstabilität erreicht werden. Es ist sogar möglich, entsprechend durch Gefriertrocknung kryokonservierte Konstrukte bereitzustellen. Es zeigte sich, dass die erfindungsgemäßen Konstrukte in einem entsprechenden Konservierungsmedium über mindestens zwei Monate im Kühlschrank gelagert werden konnten. Es zeigte sich weiterhin, dass die so erhaltenen bioartifiziellen und azellulären Konstrukte einer Kryokonservierung unterworfen werden können, womit die Lagerungsfähigkeit weiterhin gesteigert wird. Die Konstrukte können über sechs Monate gelagert werden.

Durch die gute Lagerfähigkeit und Stabilität während der Lagerung ist eine schnelle Versorgung möglich. Da die Konstrukte azelluläre Konstrukte sind, ist deren Einsatz vom Individuum unabhängig möglich. Im Gegensatz zu zellulären Konstrukten, die eine individuelle *in vitro* Herstellung erfordern, können die erfindungsgemäßen Konstrukte gelagert und schnell eingesetzt werden.

Offenbart wird (nicht unter den Schutzumfang der Ansprüche fallend) eine Druckbeaufschlagung der auf der Oberfläche aufgebrachten Mischung durch Einbringen dieser Mischung in eine rotierende Hohlform (Rotationsform), so dass sich die Zusammensetzungen auf die Oberfläche der Hohlform verteilen und ausbreiten. In einer Ausführungsform erlaubt die starke Verdünnung der Fibrinogenpräparation eine hervorragende Verteilung der Zusammensetzungen in der rotierenden Form. Die Druckbeaufschlagung erfolgt in Form von durch Rotation der Hohlform entstehende Zentrifugalkräfte (nicht unter den Schutzumfang der Ansprüche fallend).

Ein Rotationsverfahren zur Herstellung eines Gewebekonstrukts wird zum Beispiel in der WO 2013/091865 A1 beschrieben. Dort wird allerdings ein Matrixmaterial, wie Fibrinogen, zusammen mit Zellen mithilfe eines Applikators in Form einer Lanzette in die rotierende Hohlform eingebracht, wobei der Applikator während des Einbringens längs der Rotationsachse verschoben wird. Erfindungsgemäß betrifft das Verfahren die Herstellung eines azellulären Konstrukts, das heißt, die eingebrachten Zusammensetzungen sind zellfrei.

Weiterhin ist in einer Ausführungsform das Verschieben des Applikators längs der Rotationsachse nicht notwendig, da die Fibrinogen-haltige Zusammensetzung und die Thrombin-haltige Zusammensetzung allein oder als Mischung sehr wässrig sind und sich sehr gut in der rotierenden Form verteilen. Eine Applikation kann auch derart erfolgen, dass sie an einem Ende der rotierenden Form erfolgt, die Mischung verteilt sich dann gleichmäßig in der rotierenden Form, während diese Mischung durch Polymerisation aushärtet.

Das Einbringen der Reaktanten und die Ausbildung des Fibrins erfolgt dabei in einer Ausführungsform derart, dass an der Oberfläche der Hohlform mindestens 600g vorliegen, wie zum Beispiel mindestens 800g, zum Beispiel mindestens 900g.

In den bisherigen Rotationsverfahren, bei denen gleichzeitig Zellen mit eingebracht werden, konnten aufgrund des Vorliegens von Zellen nur wesentlich geringe g-Zahlen genutzt werden. So beschreibt die WO 2013/091865 A1 ein Einbringen der Zellen während der Rotation mit zwischen 100g und 600g. In den Beispielen wurden dabei g-Zahlen von 150 und 330g genannt. Gleiches gilt für eine Druckbeaufschlagung im Allgemeinen.

Offenbart wird (nicht unter den Schutzumfang der Ansprüche fallend), dass bei Durchführung der Druckbeaufschlagung durch Rotation eine wesentlich höhere Druckbeaufschlagung durch die Fliehkraft erfolgen kann, nämlich von mindestens 600g, wie mindestens 800g, z.B. mindestens 900g, wie mindestens 1000g. Dadurch können wesentlich verbesserte mechanische Eigenschaften erreicht werden.

Erfindungsgemäß erfolgt die Druckbeaufschlagung flächig auf die Oberfläche aufgebrachten Zusammensetzungen. Diese flächige Druckbeaufschlagung erlaubt die Ausbildung komplexer Konstrukte. Das heißt, komplexere Strukturen mit individueller Geometrie können entsprechend ausgebildet werden. Dabei wird ein Substrat der gewünschten Form bereitgestellt und auf die Oberfläche dieses Substrats werden die Zusammensetzungen aufgebracht. Der Vorteil der Ausbildung der flächigen Produkte auf Basis des bereitgestellten Substrats erlaubt gegenüber dem Rotationsverfahren nicht nur die Ausbildung von entsprechenden rohrförmigen Konstrukten, sondern auch die Möglichkeit, andere Geometrien bereitzustellen. Es können unterschiedlichste Geometrien ausgebildet werden, hierbei ist im Wesentlichen jede Form vorstellbar auch hinterschnittene Bereiche.

Entsprechend ist es möglich, dass die so hergestellten Strukturen bereits die gewünschten mechanischen Eigenschaften aufweisen, die bei Ausbildung von einfachen Strukturen auf einer Fläche oder im Rotationsverfahren hergestellte Strukturen durch Veränderung des Konstruktes, wie Aufschneiden, Zerschneiden der Struktur und Anpassung an eine dreidimensionale Form, verschlechtert werden.

Erfindungsgemäß erfolgt die Druckbeaufschlagung, die flächig auf die auf der Oberfläche aufgebrachten Zusammensetzungen erfolgt, mit einem relativen Druck von mindestens 100 kPa [1 bar], wie mindestens 500 kPa [5 bar], mindestens 1000 kPa [10 bar], wie mindestens 1500 [15 bar].

Durch das erfindungsgemäße Verfahren ist es möglich, Konstrukte bereitzustellen mit Wanddicken von mehr als 1 mm, wie 1,5mm und dicker, zum Beispiel mehr als 2mm. Solche Schichten sind aus dem Stand der Technik bisher nicht beschrieben. Üblicherweise konnten Schichten von maximal 0,5 mm aufgebracht werden. Vorteilhafterweise erlaubt somit das erfindungsgemäße Verfahren die Bereitstellung von bioartifiziellen, azellulären Konstrukten mit erhöhten Schichtdicken.

Erfindungsgemäß wird in einem weiteren Schritt dann das so erhaltene azelluläre bioartifizielle Konstrukt auf Fibrinbasis von der Oberfläche entfernt. Entsprechend kann die Oberfläche derart ausgebildet sein, dass ein einfaches Entfernen erfolgt. Zum Beispiel kann es sich hier um eine Teflonoberfläche oder andere geeignete und bekannte Oberflächen handeln.

In einer Ausführungsform ist es erfindungsgemäß möglich, dass die Zusammensetzungen, die Fibrinogen-haltige Zusammensetzung und die Thrombin-haltige Zusammensetzung, zusammen oder getrennt mehrfach aufgebracht werden.

Hierdurch können stärkere Dicken des Konstrukts erzielt werden.

Das Verfahren beinhaltet Ausführungsformen, bei denen mehrere Schichten aufgebracht werden. Bei diesen Schichten kann es sich um gleiche Schichten handeln. Alternativ können die Konstrukte auf Fibrinbasis auch andere nicht-Fibrin-haltige Schichten aufweisen.

In einer Ausführungsform kann dabei die im Verfahren hergestellte oberste Schicht eine Heparin-haltige Schicht sein. Dazu wird erfindungsgemäß zuerst eine Thrombin-haltige Zusammensetzung als oberste Schicht aufgebracht und anschließend eine Heparin-haltige Zusammensetzung. Dem Fachmann sind die entsprechenden Zusammensetzungen bekannt. Thrombin besitzt sowohl eine Bindungsstelle für Fibrin als auch für Heparin. Durch die Applikation von Heparin im letzten Schritt des Herstellungsprozesses wird das Heparin über das Thrombin an die Oberfläche gebunden.

Durch Ausbildung dieser Heparin-haltigen Schicht können bei Verwendung des erfindungsgemäßen Konstrukts als Gefäßprothese Blutgerinnsel vermieden werden.

Weiterhin können Schichten in dem Konstrukt vorliegen, die eine nicht-Fibrin-haltige Schicht sind, diese nicht-Fibrin-haltige Schicht kann aus anderen biologischen Materialien, die zellfrei sind, oder aus synthetischen Materialien bestehen. Diese Materialien schließen ein biologische Materialien wie andere Bestandteile der extrazellulären Matrix, z.B. Kollagen, synthetische biokompatible Materialien z.B. Polymere, Keramiken oder Metalle, auch Hybride hieraus. Auch die Einbringung von Wirkstoffen kann hier von Vorteil sein, z.B. selektive Wachstumsfaktoren, antimikrobielle Substanzen oder Mediatorstoffe wie Cytokine.

Dabei kann diese nicht-Fibrin-haltige Schicht zwischen Fibrin-haltigen Schichten angeordnet sein oder an einer Seite oder an beiden Seiten. Es wird dabei davon ausgegangen, dass durch die nicht vollständige Ausbildung des Fibrins zwischen den Schichten, ob Fibrin-haltige Schichten oder zwischen Fibrin-haltigen und nicht-Fibrin-haltigen Schichten, eine Verbindung derart erfolgt, dass die Schichten ineinander greifen.

Erfindungsgemäß weisen die aufgebrachten Schichten nach Druckbeaufschlagung eine Dicke von mindestens 1mm auf, wie mindestens 1 ,5mm, zum Beispiel mindestens 2mm.

In einer Ausführungsform handelt es sich bei dem Fibrinogen um eines aus Plasma isoliertes Fibrinogen. In einer weiteren Ausführungsform handelt es sich bei dem Thrombin um eines aus der gleichen Menge Plasma isoliertes Thrombin.

Das eingesetzte Fibrinogen und/oder Thrombin kann dabei ein autologes, allogenes oder xenogenes Material sein. Bevorzugt wird autologes oder allogenes Material verwendet, um mögliche Reaktionen im Körper des Empfängers zu vermeiden.

Das erfindungsgemäße Verfahren erlaubt die Bereitstellung von bioartifiziellen, azellulären Konstrukten, wobei diese Herstellung schneller und deutlich einfacher ist, als eine bei denen Zellen bereits während des Herstellungsverfahrens integriert werden. Aufgrund des zellfreien Arbeitens ist es weiterhin möglich, Matrixstrukturen in dem Konstrukt auszubilden, die einer wesentlich höheren mechanischen Belastung ausgesetzt werden können, um so die biomechanischen Eigenschaften des Konstrukts zu verbessern. Es wird davon ausgegangen, dass dieses durch die weitaus stärkere Verdichtung der Fibrinmatrix möglich ist.

Es ist somit ein Einsatz während der Operation möglich, azelluläres autologes Material von den Patienten zu isolieren, das Konstrukt erfindungsgemäß herzustellen und dieses anschließend gleich in dem Empfänger einzubringen.

Weiterhin ist durch den Entfall des primären Einsatzes von Zellen auch das Herstellungsverfahren insgesamt einfacher, die entsprechenden Matrizes können stärker verdichtet werden, als dieses mit den darin enthaltenen Zellen möglich wäre. Bei den hohen erfindungsgemäß möglichen Kräften würden bei Zellen die Gefahr einer Zellschädigung oder eines Zelltods bestehen.

Das erhaltene Konstrukt mit seiner Fibrinmatrix weist weiterhin wesentlich verbesserte biomechanische Eigenschaften gegenüber den bekannten Gewebekonstrukten auf. Dieses gilt sowohl für die Scherkräfte als auch für die weiteren einwirkenden Kräfte.

Mithilfe des erfindungsgemäßen Verfahrens können somit auch Konstrukte bereitgestellt werden, die in biomechanisch stark beanspruchtem Gewebe eingesetzt werden können, wie zum Beispiel Knorpel oder Herzklappensegel.

Durch den primären Verzicht von Zellen kann weiterhin ein besser lagerungsfähiges Konstrukt bereitgestellt werden. Tatsächlich zeigte sich, dass neben der bekannten Lagerung in üblichen Lagermedien, zum Beispiel Glutaraldehyd, die erfindungsgemäßen Konstrukte auch einer Gefriertrocknung zugänglich sind. Durch die Dehydrierung bei der Herstellung und insbesondere bei Gefriertrocknung bzw. Kryokonservierung kann die Matrix lagerstabil ausgebildet werden. Auch nach Lagerung zeigen die Konstrukte die gewünschte Stabilität auf.

Weiterhin ist mithilfe des Verfahrens der Gefriertrocknung nicht nur eine theoretisch unbegrenzte und unkomplizierte Lagerungsfähigkeit möglich, sondern es kann gleichzeitig über den damit verbundenen Wasserentzug auch von einer noch weiter verbesserten biomechanischen Belastbarkeit ausgegangen werden.

Darüber hinaus können Konstrukte bereitgestellt werden, die bei Einsatz entsprechend in ihrer gewünschten Größe "angepasst" werden. Aufgrund der guten Lagerungsfähigkeit und der einfachen Herstellung ist es möglich ein Endlosmaterial bereitzustellen, das entsprechend den Anforderungen angepasst werden kann.

Darüber hinaus erlaubt das erfindungsgemäße Verfahren die Bereitstellung von Konstrukten mit größerer Wanddicke, dieses führt entsprechend zu verbesserten biomechanischen Eigenschaften.

Erfindungsgemäß ist es auch möglich durch Variation der Druckbeaufschlagung bei Aufbringen von mehreren Schichten eines Konstrukts gebildet aus mehreren Schichten diesen Schichten unterschiedliche mechanische Eigenschaften zu verleihen. Durch unterschiedlich starke Verdichtung und Zusammensetzung der Komponenten können entsprechende unterschiedliche Eigenschaften der Schichten in einem Konstrukt generiert werden.

Erfindungsgemäß können während des Verfahrens weitere nicht-zelluläre Komponenten und Substanzen hinzugefügt werden, die dann in das Konstrukt integriert werden oder an der Oberfläche des Konstrukts vorliegen. Solche Substanzen können Plasmakomponenten sein, diese können als autologe und/oder allogene oder xenogene und/oder synthetische Plasmakomponenten eingesetzt werden. Weiterhin können weitere stabilisierende Substanzen, zum Beispiel autologen, allogenen, xenogenen oder synthetischen Ursprungs eingesetzt werden, um biomechanische Eigenschaften weiter zu verändern.

Zusätzlich können auch andere Substanzen genutzt werden, zum Beispiel solche zur Vermeidung/Verringerung von Infektionen oder zur Induktion von Gewebeeigenschaften wie Induktion von Wachstum, Regeneration usw. Ein solcher Einsatz von Substanzen war bisher nur sehr begrenzt möglich, da diese gegebenenfalls toxischer Eigenschaften auf die im Gewebekonstrukt vorliegenden Zellen aufweisen können.

Das erfindungsgemäße Konstrukt kann weiterhin andere Substanzen zur Verbesserung der Gewebeeigenschaften enthalten, wie zum Beispiel Heparin, Wachstumsfaktoren, antimikrobielle Substanzen aber auch toxische Substanzen.

Offenbart wird (nicht unter den Schutzumfang der Ansprüche fallend), dass bei Druckbeaufschlagung durch Rotation das Verfahren derart durchgeführt, dass mithilfe eines fest positionierten Applikators die Zusammensetzungen bei Rotation in das System eingebracht werden. Diese verteilen sich gleichmäßig an den Wänden und Polymerisieren aus. In einer Alternative ist der Applikator einer, der in der Form beweglich angeordnet ist, so dass durch hinein- und hinausfahren die Fibrinogen- und die Thrombinlösung aufgebracht werden. Dabei können die beiden Lösungen möglichst spät miteinander in Kontakt kommen und vermischt werden, damit die Reaktion erst nach Aufbringen erfolgt.

Dabei wird in einer Ausführungsform des Verfahrens über den ersten Zeitraum keine Flüssigkeit aus dem System geführt. So ist dieser erste Zeitraum zum Beispiel ein Zeitraum von maximal fünf Minuten, wie maximal drei Minuten, wie maximal zwei Minuten nach Einbringen der Zusammensetzungen. Anschließend wird die überschüssige Flüssigkeit abgeführt.

In einer anderen Ausführungsform weist die Form Bohrungen auf, so dass Flüssigkeit durch diese Bohrungen abgeführt werden kann. Diese Bohrungen, zum Beispiel mit einem Durchmesser zwischen 0,1 mm und 1mm, sind in einer Ausführungsform mit einem semipermeablen Material, bevorzugt an der Außenseite der Form, abgeschlossen. Dieses semipermeable Material erlaubt die Abtrennung von Flüssigkeit, während das Fibrinogen und das Thrombin sowie das gebildete Fibrin in der Form verbleiben. Durch diese Drainage ist es möglich, ausreichend Flüssigkeit abzuziehen und dem Konstrukt die notwendige Stabilität zu verleihen.

In einem weiteren Aspekt wird erfindungsgemäß ein bioartifizielles, azelluläres Konstrukt auf Fibrinbasis erhältlich gemäß dem erfindungsgemäßen Verfahren bereitgestellt. Dieses mithilfe des erfindungsgemäßen Verfahrens erhältliche azelluläre, bioartifizielle Konstrukt weist gegenüber den bekannten Gewebekonstrukten verschiedenste Vorteile auf:
Das erfindungsgemäße Konstrukt weist hervorragende biomechanische Eigenschaften auf, so dass auch ein Einsatz bei stark beanspruchten Geweben, wie Knorpel oder Herzklappensegel möglich ist.

Die Konstrukte weisen aufgrund des Verzichts auf Zellen eine verbesserte Lagerungsfähigkeit auf, insbesondere kann eine Gefriertrocknung des Konstrukts erfolgen. Erfindungsgemäß können somit gefriergetrocknete bioartifizielle, azelluläre Konstrukte auf Fibrinbasis bereitgestellt werden. Erfindungsgemäß umfasst ein erfindungsgemäßes Verfahren damit in einer Ausführungsform weiterhin den Schritt der Gefriertrocknung des erhaltenen Konstrukts.

Das Konstrukt ist deutlich einfacher herzustellen und aufgrund des Verzichts von Zellen ist die Herstellung hiervon und der Einsatz auch unter Zulassungsbedingungen wesentlich vereinfacht. Insbesondere erlaubt das erfindungsgemäße Konstrukt auf Fibrinbasis die Individualisierung der Länge und des Durchmessers hiervon. Es können vorgefertigte Konstrukte als Medizinprodukte bereitgestellt werden, um dann individuell angepasst dem Empfänger eingesetzt zu werden.

Das erfindungsgemäße Konstrukt zeichnet sich durch eine stärkere Verdichtung im Vergleich zu herkömmlichen Gewebekonstrukten aus, die die biomechanischen Eigenschaften verbessern.

Weiterhin können Konstrukte bereitgestellt werden, die eine wesentlich stärkere Dicke aufweisen, zum Beispiel Hohlkörper mit Wanddicken von >2mm. Darüber hinaus können die erfindungsgemäßen Konstrukte Beschichtungen, wie Heparinbeschichtungen aufweisen, die das Ausbilden von Thromben verhindern.

Die erfindungsgemäßen bioartifiziellen, azellulären Konstrukte können dabei individualisierte Formen unterschiedlichster Geometrien aufweisen.

Darüber hinaus zeigte sich überraschend, dass solche Konstrukte, wenn sie als Gefäßprothese eingesetzt werden, mindestens die gleich guten Eigenschaften aufweisen, wie *in vitro* generierte Gefäßprothesen, die entsprechende, im Gefäß vorhandene Zellen enthalten, wie glatte Muskelzellen, Endothelzellen, usw. Tatsächlich zeigte sich, dass die Gefäßprothesen auch ohne Zellen eingesetzt werden können und Zellen einsprossen und sich entsprechende Strukturen ausbilden. Diese Ausbildung erfolgt dabei derart, dass die Strukturen den gleichen Aufbau haben wie die natürlich vorkommenden Strukturen, zum Beispiel bei Gefäßen Strukturen mit entsprechender Anordnung von Endothelzellen, glatten Muskelzellen usw. Das Sprossen der Zellen geht von außen nach innen, der Durchmesser der Gefäße wird aber nicht beschränkt. Das heißt, die Zellen wachsen aus dem umgebenden Gewebe in die Konstrukte aber nicht in das Lumen des Hohlkörpers. Es zeigte sich, dass sechs Wochen nach Implantation die erfindungsgemäßen Konstrukte nicht mehr unterscheidbar waren zu normalen Gefäßstrukturen. Das Fibrin der Struktur war nach einigen Monaten nicht mehr nachweisbar, so dass die erfindungsgemäßen Konstrukte nach Implantation vollständig abgebaut werden.

Die erfindungsgemäßen Konstrukte können dabei je nach Herstellungsverfahren als Patch oder als rohrförmiger Körper ausgebildet sein.

Diese erfindungsgemäßen Konstrukte können dann in Tissue Engineering eingesetzt werden, zum Beispiel auch zur *in vitro* Präkonditionierung. So ist zum Beispiel ein Einsatz als Knorpelersatz möglich, das heißt, es werden kleine Stückchen des Fibrinkonstrukts in die betreffenden Bereiche im Körper eingebracht und es findet *in vivo* eine Besiedlung durch die Chondrozyten und Chondroblasten statt.

Weitere Anwendungsgebiete der erfindungsgemäßen bioartifiziellen, azellulären Konstrukte auf Fibrinbasis neben der Gefäßprothese sind alle Arten von Implantaten, wie Knorpelersatz, aber auch als Matrix für die in vitro Herstellung. Darüber hinaus können die erfindungsgemäßen Konstrukte auch für biomechanisch stark beanspruchte Gewebe eingesetzt werden, wie Knorpel oder Herzklappensegel.

In einer Ausführungsform ist das bioartifizielle, azelluläre Konstrukt auf Fibrinbasis dabei eines erhältlich durch Verwendung von autologen Materialien.

Schließlich richtet sich die Anmeldung auf die erfindungsgemäßen bioartifiziellen, azellulären Konstrukte zur Verwendung in Verfahren als Implantat, Knorpelersatz, sowie die Verwendung der Konstrukte als Matrix für *in vitro* Herstellung von Gewebeersatz oder Gefäßprothese, genauso wie für Herzklappensegel, Gewebe zur Rekonstruktion kardiovaskulärer Strukturen wie z.B. dem Vorhof- oder Ventrikelseptum oder dem links- oder rechtsventrikulären Ausflusstrakt. Auch Strukturen des Hörapparates, der Haut oder innerer Organe lassen sich damit generieren. Die erfindungsgemäßen Konstrukte können auch zur *in vitro* Präkonditionierung, zum Beispiel bei Gewebetransplantation bei Verbrennungspatienten usw. eingesetzt werden. Ein Aspekt der vorliegenden Erfindung richtet sich somit auf ein azelluläres Konstruktes zur Verwendung in einem Verfahren der Transplantation in ein Individuum, mit dem Schritt des Implantierens eines erfindungsgemäßen bioartifiziellen und azellulären Konstrukts auf Fibrinbasis.

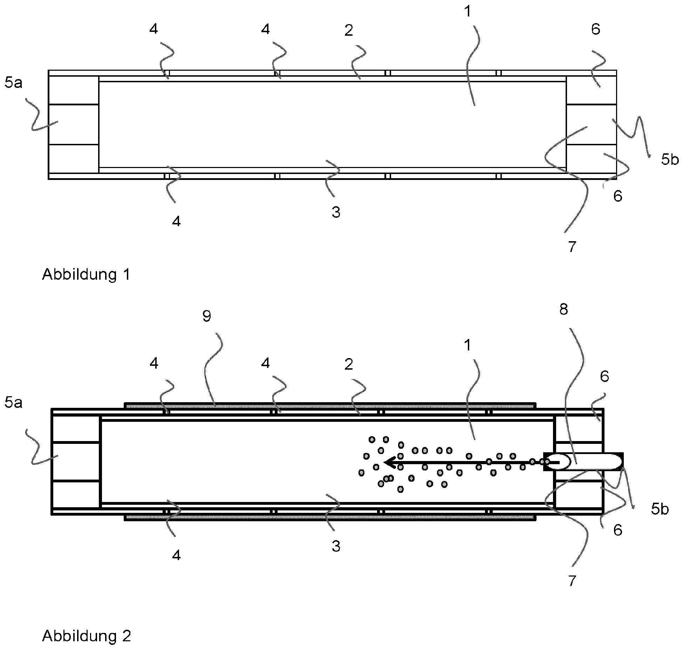

In der Abbildung 1 (nicht unter den Schutzumfang der Ansprüche fallend) wird eine verwendbare Rotationsform 1 dargestellt. Die Rotationsform 1 weist dabei eine äußere Hülle 2, die zum Beispiel in Form eines Metallrohrs ausgestaltet sein kann, auf. Die innere Form 3 ist derart ausgebildet, dass das Fibrinkonstrukt nicht daran haftet. Zum Beispiel kann die innere Form 3 durch zwei Teflonhalbschalen ausgebildet sein. Die Größe der inneren Form 3 bestimmt den äußeren Durchmesser des herzustellenden Fibrinkonstrukts.

In einer Ausführungsform kann die äußere Hülle 2 mindestens eine Bohrung 4 aufweisen. Diese Bohrungen 4 in der äußeren Hülle 2 der Form 1 erlauben, die Flüssigkeit aus der Form 1 bei Rotation herauszuführen.

Weiterhin weist die Rotationsform 1 an beiden Enden eine Manschette 5a, 5b, auch als Cuff bezeichnet, auf. Diese Manschette ist dabei derart ausgebildet, dass sie im mittleren Bereich durchlässig ist für Flüssigkeiten und über einen gesamten Bereich für Gase. Die Manschetten 5a, 5b mit mehreren Abschnitten 6, 7 bestimmen den Flüssigkeitsspiegel der eingebrachten Fibrinogen- und Thrombinsuspension.

In der Abbildung 2 (nicht unter den Schutzumfang der Ansprüche fallend) ist das Einbringen der Fibrinogen und Thrombin enthaltenden Lösung dargestellt. Dazu wird mithilfe eines Applikators 8 durch die Manschette 5 die Lösung beziehungsweise die Suspension appliziert. Dabei sind zum Beginn des Rotierens die Bohrungen 4 abgedeckt mit einer Abdeckung 9. Diese Abdeckung 9 kann zum Beispiel in Form einer lösbaren Folie etc. sein. Alternativ können die Bohrungen mit einem semipermeablen Material als Abdeckung 9, wie einem Vlies oder ähnliches, abgedeckt sein. Dieses semipermeable Material erlaubt ein Abtrennen der Flüssigkeit, während das Fibrinogen und das Thrombin in der Form verbleiben.

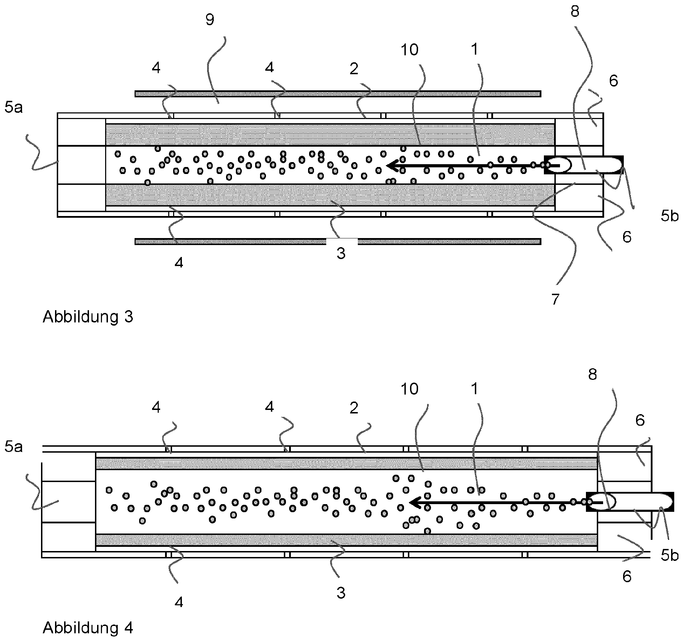

In der Abbildung 3 (nicht unter den Schutzumfang der Ansprüche fallend) wird die Fibrinogen- und Thrombin-haltige Lösung über den Applikator 8 weiter durch die Manschette 5 appliziert. Die Abdeckung 9 wurde gegebenenfalls von den Bohrungen 4 entfernt, um ein Abziehen der Flüssigkeit zu ermöglichen. Bei Verwendung eines semipermeablen Materials verbleibt die Abdeckung auf der Außenseite. Die Fibrinogensuspension enthaltend das Thrombin ist üblicherweise sehr wässrig ausgebildet, durch die geringe Konzentration des aktivierenden Thrombins findet eine langsame Polymerisierung statt und erlaubt eine gleichmäßige Verteilung in der Rotationsform. Die Abdeckung 9, wenn notwendig, wird zum Beispiel geöffnet, wenn die Form zur Hälfte gefüllt ist. Das Fibrinkonstrukt 10 bildet sich an der inneren Form 3 aus. Wenn die Abdeckung 9 ein semipermeables Material ist, verbleibt dieses auf der Außenseite.

Die Fibrinogen-/ Thrombin-haltige Lösung wird über den Applikator 8 durch die Manschette 5 weiter appliziert, wie in der Abbildung 4 (nicht unter den Schutzumfang der Ansprüche fallend) dargestellt. Durch die Bohrungen 4 kann die überschüssige Flüssigkeit entweichen, bereits polymerisiertes Fibrin 10 wirkt dabei als Filter und hält weiteres Fibrin und Fibrinogen zurück, so dass nur Flüssigkeit, das heißt das wässrige Lösungsmittel, durch die Bohrungen 4 abgeführt werden. Die Flüssigkeit wird durch die Rotation aus der Form herausgepresst, während das polymerisierende Fibrin durch die Zentrifugalkräfte verdichtet.

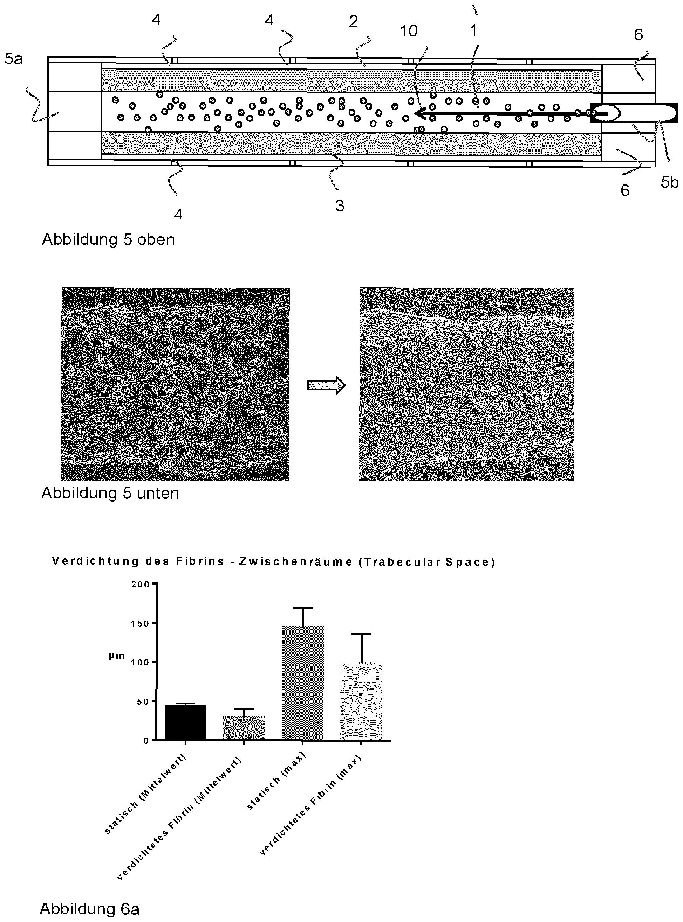

In der Abbildung 5 (nicht unter den Schutzumfang der Ansprüche fallend) ist die Verdichtung des polymerisierenden Fibrinkonstrukts weiter dargestellt. Abbildung 5 oben zeigt die während des Rotationsverfahrens weiter eingebrachte Thrombin und Fibrinogen enthaltende Suspension unter Ausbildung des Fibrinkonstrukts. Diese Suspension wird so lange eingebracht, bis diese den von der Manschette 5 nur Gas durchlassenden Bereich 6 ausgefüllt ist.

In der Abbildung 5 unten ist die Verdichtung des Fibrinnetzes 10 dargestellt. Die Zentrifugalkräfte verdichten das polymerisierte Fibrin an der Wand der Form 1, während Flüssigkeit aus der Form gepresst wird. Die Verdichtung des Fibrinnetzes ist auf der rechten Abbildung zu erkennen.

Das so ausgebildete Konstrukt kann dann aus der Rotationsform 1 als Hohlkörper herausgenommen werden. Ein Protokoll zur Durchführung des (nicht unter den Schutzumfang der Ansprüche fallend) Verfahrens mit einer solchen Rotationsform ist in den Beispielen ausgeführt.

Im Folgenden wird die Erfindung anhand von Beispielen näher erläutert ohne hierauf beschränkt zu sein:
Beispiel 1 (nicht unter den Schutzumfang der Ansprüche fallend)
   Protokoll zur Generierung eines bioartifiziellen Gefäßersatzes mittels einer Rotationsform (Centrifugal casting):
   1. Präzipitation des Fibrinogens aus Plasma eines Spenderbluts mittels eines Gefrier-/ Auftau-Zyklus (Kryopräzipitation) gemäß bekannter Verfahren. Alternativ mittels Zugabe von Ethanol (Ethanolpräzipitation). Üblicherweise werden aus 50 ml Plasma 8-10 ml Fibrinogenpräparation präzipitiert.
   2. Resuspension des separierten Fibrinogens im abgetrennten Plasma in geringer Konzentration (10 bis maximal 20 mg/ml).
      Das Fibrinogen liegt im Plasma relativ stark verdünnt vor (10 - 20 mg/ml), so dass nun eine vergleichsweise hoch konzentrierte Thrombinkomponente dazugegeben werden kann, um das Fibrinogen zur Aushärtung zu bringen. Alle bisherigen Ansätze im Stand der Technik versuchten über die Konzentration des Thrombins die Geschwindigkeit der Aushärtung des Fibrinogens zu steuern. Es zeigte sich aber, dass der hier verwendete Ansatz vorteilhaft ist im Hinblick auf eine homogenere Durchmischung und Verteilung des Gemisches in der rotierenden Form.
   3. Applikation der Fibrinogenpräparation simultan mit einer Thrombinlösung im Verhältnis 6:1 in die rotierende Form. Die Thrombinkomponente besteht pro 1 ml aus:
      - 850 Einheiten Thrombin in 850 µl CaCl-Lösung (40 mmol/l)
      - 100 µl CaCl (1 M)
      - 50 µl Aprotininlösung (250.000 KIU/ ml)
   4. Aufgrund der starken Verdünnung der Fibrinogenpräparation ist diese Lösung sehr wässrig und wird langsam fest und verteilt sich daher sehr gut in der rotierenden Form. Daher kann in diesem Ansatz die Applikation von einem Ende der rotierenden Form erfolgen. Es ist auch möglich Konstrukte langer Segmente von mindestens 20 cm und von Segmenten mit einem inneren Durchmesser von 2 mm herzustellen.
   5. Applikation der Lösungen also von einem Ende. Die Form rotiert bereits initial mit 10.000-15.000 rpm (je nach Durchmesser der Form bis zu 900g). Zunächst werden die Bohrungen in der Form, über die überschüssige Flüssigkeit während der Zentrifugation aus der Form heraus gepresst wird, verschlossen. Die Form wird zu 40% ihres Volumens gefüllt.
   6. Zentrifugation bei 10.000-15.000 rpm für 2 min. Dann werden die Bohrungen in der Form wieder geöffnet. Gegebenenfalls muss die Form dazu kurz angehalten werden, um die Abdeckung der Bohrungen zu entfernen.
   7. Danach weitere Applikation der Fibrinogen- und Thrombinlösungen (Verhältnis 6:1) bei Rotation der Form mit 10.000-15.000 rpm.
   8. Applikation von insgesamt 8-10 ml der Fibrinogenpräparation (150-200 mg Fibrinogen) separiert aus 50 ml Plasma.
   9. Nach vollständiger Applikation (Dauer insgesamt ca. 5 Minuten) weitere Rotation der Form für weitere 15 Minuten.
   10. Für eine Heparinbeschichtung der Oberfläche werden dann 1 ml Thrombinlösung in die weiter rotierende Form appliziert. Die Lösung besteht aus:
      - 850 Einheiten Thrombin in 850 µl CaCl-Lösung (40 mmol/l)
      - 150 µl CaCl (1 M)
   11. Nach weiterer Rotation der Form für 5 min. Applikation von 1 ml Heparinlösung (5.000 Einheiten Heparin pro ml).
   12. Weitere Rotation der Form für weitere 15 Minuten.
   13. Anhalten der Rotation und Entnahme des Gefäßes aus der Form.

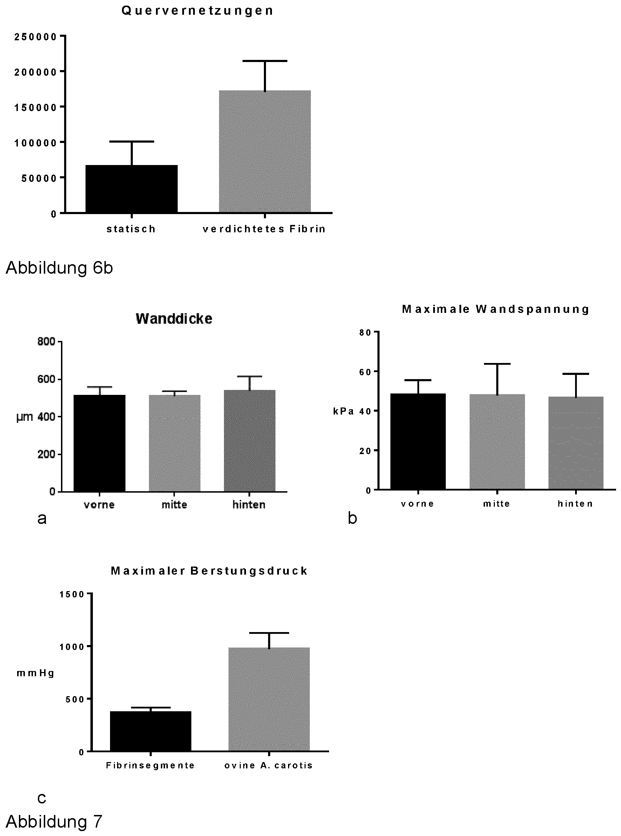

   In der Abbildung 7 sind Eigenschaften des so hergestellten Konstrukts gezeigt. Durch den temporären Verschluss der Bohrungen wirkt bereits appliziertes und an der Wand der Form polymerisiertes Fibrin als Filter für weiteres Fibrinogen, das nachfolgend appliziert wird. Mit dieser Technik werden eine gleichmäßige Verteilung des Fibrins (a) und eine gleichmäßige biomechanische Stabilität der Wand (b) erreicht. Bei dieser Technik können bis zu 250 mg Fibrinogen in eine rotierende Form mit 100 mm Länge und 6 mm Durchmesser appliziert werden. Damit können Fibrinsegmente generiert werden, die einen maximalen Berstungsdruck (Burst strength) von etwa 400 mmHg aufweisen (c).
Bespiel 2 (nicht unter den Schutzumfang der Ansprüche fallend)
   Alternativprotokoll
   1. Aufbau der Rotationsform aus einer äußeren Messinghülse, in die zwei Halbschalen aus Teflon eingepasst werden. Die beiden Enden werden mit jeweils einem Cuff aus Teflon, der jeweils eine zentrale Bohrung mit 3 mm Durchmesser hat, eingeengt um einen Verlust des Fibrins an den Enden zu vermeiden. Aufkleben von Leukopor (BSN Medical GmbH, Hamburg) von außen auf das Messingrohr der Form.
   2. Die so präparierte Form wird in den Konnektor eines Elektromotors geschoben, mit dem die Form rotiert wird.
   3. Der Applikator wird aus einem Stahlrohr mit einem Durchmesser von 2,5 mm und zwei Silikonschläuchen mit einem Durchmesser von 1 mm zusammengesteckt, so dass das Ende der Schläuche etwa 2 mm über das Ende des Stahlrohrs hinausragen. Das andere Ende des Silikonschlauches wird über Luerlock-Konnektoren jeweils an eine Spritzenpumpe angeschlossen.
   4. Fixierung des Applikators auf einem justierbaren elektrisch betriebenen Schlitten, so dass das Ende des Applikators entsprechend der Länge der Form freihängt. Das freihängende Ende kann dann mit dem elektrisch betriebenen Schlitten in die Form hineingefahren werden.
   5. Aufziehen der Fibrinogenpräparation und der Aktivatorlösung in jeweils eine Spritze, die an die Silikonschläuche im Applikator angeschlossen werden. Verwendung einer aufgereinigten Fibrinogenpräparation (s. Beispiel 3) mit einer Konzentration von 40-60 mg/ml. Die Konzentration des Thrombins in der Aktivatorlösung beträgt 100 Einheiten pro ml.
   6. Rotation der Form mit 5.000-8.000 Umdrehungen pro min. (rpm), während mit einem Vorschub von 2 (1-3) mm pro Sekunde der Applikator von einem Ende her in die Form hineingefahren wird. 7. Mehrmalige Wiederholung des Schrittes 6.
Beispiel 3
   Aufreinigung/ Waschen von Fibrinogen
   1. Einfrieren von gewonnenem Plasma bei -20°C für mindestens 24 Stunden.
   2. Auftauen des Plasmas bei Raumtemperatur.
   3. Zentrifugation des vollständig aufgetauten Plasmas bei 200-300 g für 3 Minuten.
   4. Vollständiges Abnehmen des überstehenden Plasmas und Resuspension des Fibrinogen-/Proteinpellets aus 50 ml Plasma in 20 ml Aqua ad iniectabilia, das für mindestens 24 Stunden bei -20 °C eingefroren.
   5. Die eingefrorene Fibrinogen-/ Proteinsuspension wird bei Raumtemperatur aufgetaut. Die vollständig aufgetaute Suspension wird bei 200-300 g für 3 Minuten zentrifugiert.
   6. Vollständige Abnahme des Überstandes und Resuspension des noch wässrigen Fibrinogenpellets im Wasserbad bei 37°C ohne weitere Zugabe von Flüssigkeit.
Beispiel 4

### Protokoll zur Generierung eines Gewebepatches im Druckbehälter

Die Fibrin-haltige und die Thrombin-haltige Lösung werden simultan unter gleichzeitiger Durchmischung in einem entsprechenden Applikator in eine Form gegeben, so dass das Verhältnis von Thrombin (Einheiten pro ml) zu Fibrinogen (mg pro ml) in einem Bereich von 1:1 bis 10:1 liegt.

Größe und Form der Form sind variabel. Die Form erlaubt die Zufuhr von Druckluft bis zu einem Druck von 1500 kPa [15 bar]. Eine poröse Grundplatte der Form ermöglicht das Entweichen von Flüssigkeit während der Durckbeaufschlagung.

Die Druckbeaufschlagung erfolgt in einer Ausführungsform während der Polymerisierung des Fibrins. In einer Ausführungsform erfolgt die Druckbeaufschlagung im Wesentlichen nach der Polymerisierung des Fibrins.

Die Druckbeaufschlagung erfolgt mittels Druckluft flächig auf die Oberfläche der aufgebrachten Zusammensetzungen, mit einem relativen Druck von mindestens 100 kPa [1 bar], wie mindestens 500 kPa [5 bar], mindestens 1000 kPa [10 bar], wie mindestens 1500 kPa [15 bar] für 10 Minuten wie mindestens 20 Minuten wie mindestens 60 Minuten.

Nach der Druckbeaufschlagung wird die fertige Fibrinmatrix von der Grundplatte entfernt.

### Beispiel 5 (nicht unter den Schutzumfang der Ansprüche fallend)

Zur Lagerung werden die Fibrinsegmente dehydriert. Dies kann durch Kryokonservierung, Gefriertrocknung oder Vakuumtrocknung oder einfache Trocknung in trockener Luft erfolgen.

Beispielprotokoll für die Dehydrierung eines Fibrinsegments in trockener Luft:
1. Das in einer Rotationsform generierte Fibrinsegment wird auf einen Trokar (im Beispiel 3 mm Durchmesser) aufgefädelt.
2. Trokar und Fibrinsegment werden aufrecht in einen Behälter gestellt, an dessen Boden 20 g Salz gefüllt sind.
3. Wechsel des mit Salz befüllten Behälters alle 24 Stunden.
4. Dehydrierung über 72 Stunden.
5. Lagerung in einem sterilen Behältnis (nicht mit Salz befüllt) bei Raumtemperatur.

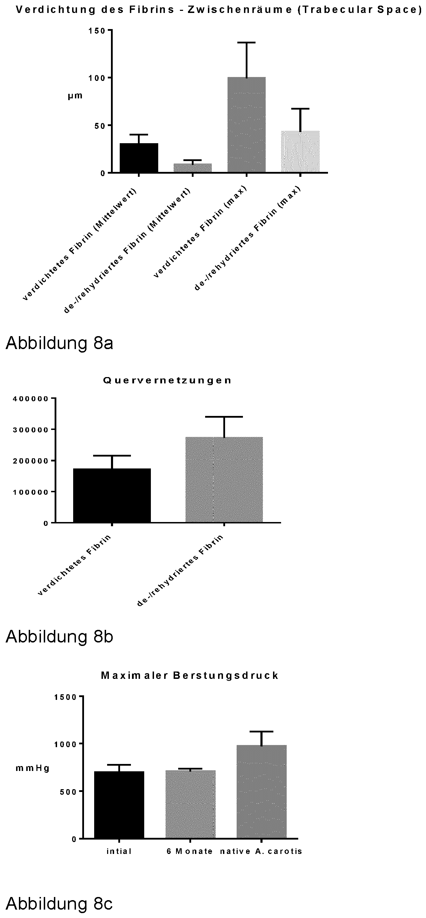

Für die Implantation werden die dyhydrierten Fibrinsegmente dann in physiologischer Kochsalzlösung rehydriert. Alternativ kommen auch andere Flüssigkeiten wie Kalziumchloridlösung in Betracht. Durch die Dehydrierung kommt es zu einer weiteren Verdichtung des Fibrins mit einer erheblichen Verbesserung der biomechanischen Eigenschaften, die nach Rehydrierung nicht oder nur teilweise wieder verloren gehen (Abbildung 8). Die Dehydrierung/ Trocknung des Fibrins führt zu einer weiteren Verdichtung des Fibrins. Die Analyse der Fibrinstruktur anhand histologischer Schnitte zeigte, dass nach Dehydrierung und nachfolgender Rehydrierung die Zwischenräume (Trabecular space) zwischen den Fibrinfibrillen signifikant abgenommen hat sowohl was die Größe der Zwischenräume im Mittel als auch deren maximale Größe angeht (a). Gleichzeitig hat die Zahl der Querverbindungen im Vergleich zu verdichtetem Fibrin, das nicht dehydriert worden ist signifikant zugenommen (b).

Die De-/ Rehydrierung führt dementsprechend auch zu einer weiteren Zunahme der Stabilität der Segmente. Aus 250 mg Fibrinogen können damit Fibrinsegmente mit einem Berstungsdruck (Burst strength) von etwa 700 mmHg hergestellt werden (c).

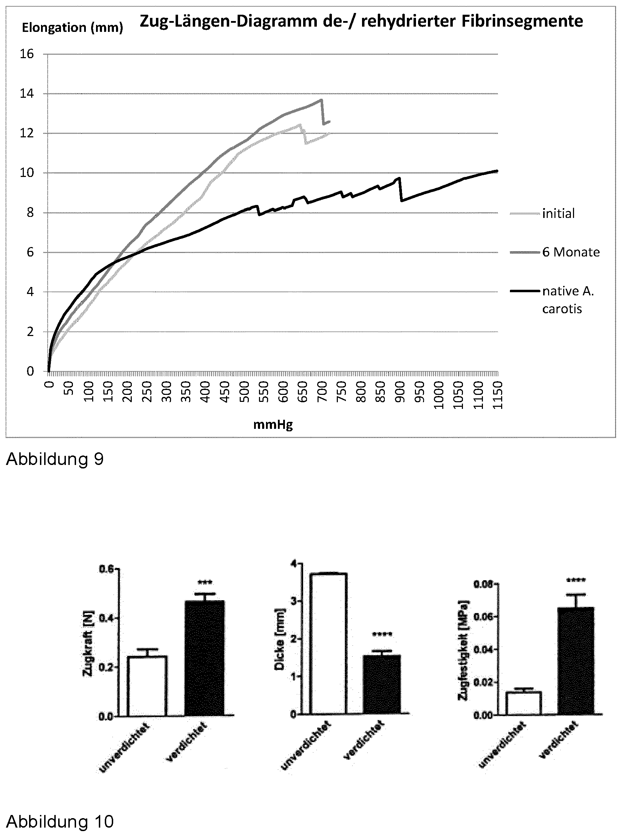

Entsprechend behandelte Fibrinsegmente weisen im Druckbereich bis 200 mmHg sogar vergleichbare biomechanische Eigenschaften hinsichtlich der Elastizität auf wie eine native Arterie. Bislang konnten wir zeigen, dass dehydrierte Fibrinsegmente für mindestens 6 Monate bei Raumtemperatur gelagert werden können (Abbildung 9). Eine deutliche längere Lagerbarkeit bei 4°C ist zu erwarten.

Die Dehydrierung und nachfolgende Rehydrierung führt zu einer Verbesserung der biomechanischen Eigenschaften der Fibrinsegmente. Bis zu einem Druck von 200 mmHg weisen diese Fibrinsegmente sogar eine vergleichbare Elastizität auf, wie eine native A. carotis. Diese Eigenschaften bleiben auch nach sechsmonatiger Lagerung bei Raumtemperatur erhalten, siehe Abbildung 9.

Die Besiedlung einer azellulären Trägerstruktur mit autologen (=körpereigenen) Zellen des Empfängers hat sich in den letzten Jahrzehnten als ein Grundprinzip des Tissue Engineerings etabliert. Damit soll erreicht werden, dass die Implantate vom Organismus als körpereigen erkannt werden und dem körpereigenen Remodeling unterliegen. Bei kardiovaskulären Implantaten soll durch die Besiedlung der Oberfläche mit Endothelzellen zudem die Thrombogenität des Implantats verringert werden. In einer in vivo-Studie wurden nicht besiedelte azelluläre Fibrinsegmente als Ersatz der A. carotis im Schaf implantiert. Bei Explantation der Segmente nach 6 Monaten waren alle Implantate durchgängig. Die histologische Aufarbeitung der explantierten Fibrinsegmente zeigte, dass diese nach Implantation in vivo einem ausgeprägten Remodeling unterliegen. Aus dem umgebenden Gewebe wandern Zellen (unter anderem Endothelzellen und glatte Muskelzellen) in das Fibrin ein und ordnen sich entsprechend dem Aufbau einer Gefäßwand an. Das Lumen wurde durch eine Endothelschicht ausgekleidet. Das Fibrin wird durch andere Matrixproteine wie Kollagen ersetzt. Diese Ergebnisse zeigen, dass es keiner Besiedlung der Fibrinsegmente vor seiner Implantation als Gefäßersatz bedarf. Zur Verringerung der Thrombogenität wird lediglich Heparin an der luminalen Oberfläche gebunden.

Das auf die Besiedlung der Fibrinsegmente verzichtet werden kann bietet mehrere Vorteile:
- Die Fibrinsegmente können leichter hergestellt werden, da die sonst nötigen Besiedlungsschritte entfallen.
- Die Fibrinsegmente nicht mehr individualisiert, d.h. sie werden nicht mehr individuell für einen Empfänger hergestellt.
- Die azellulären Fibrinsegmente können gelagert werden.

Im Vergleich mit dem derzeitigen Stand der Technik muss letzterer in zwei Gruppen unterschieden werden:
- Synthetische Gefäßprothesen (z.B. aus den Materialien Dacron oder Polytetrafluorethylen).
- Besiedelte bioartifizielle Gefäßersatzstücke.

Gegenüber den synthetischen Gefäßprothesen bieten die entwickelten azellulären Fibrinsegmente den Vorteil, dass sie nach Implantation in vivo einem Remodeling also den körpereigenen Umbau- und Reparaturmechanismen unterliegen. Damit kann im Idealfall ein Langzeitverlauf wie der einer nativen Arterie erwartet werden. Der große Vorteil gegenüber besiedelten bioartifiziellen Gefäßersatzstücken ist die Lagerbarkeit. Auf diese Weise können Gefäßersatzstücke auf Vorrat produziert und vor Ort gelagert werden. Ein entscheidender Vorteil im Hinblick auf eine klinische Nutzung.

### Beispiel 6 (nicht unter den Schutzumfang der Ansprüche fallend) Vergleich statisches Konstrukt und erfindungsgemäßes Konstrukt

Die biomechanische Stabilität wurde mit Hilfe der Software BoneJ / ImageJ, www.bonej.org untersucht. Die Untersuchungen wurden an histologischen Schnitten durchgeführt. Die Ergebnisse sind in Figur 6 dargestellt. Die Fliehkräfte in der rotierenden Form führen zu einer Verdichtung des applizierten Fibrins (verdichtetes Fibrin) im Vergleich zu einer Fibrinmatrix, die unter statischen Bedingungen generiert wurde (statisch). Die Zwischenräume zwischen den Fibrinfibrillen (Trabecular Space) werden sowohl im Mittel als auch in der maximalen Größe kleiner (a). Das führt zu einer Zunahme der Quervernetzung zwischen den Fibrillen (b).

### Beispiel 7 (nicht unter den Schutzumfang der Ansprüche fallend) Biomechanische Eigenschaften von Fibrin-Patches

Die gemäß Beispiel 2 hergestellten Fibrin-Patches wurden auf ihre biomechanischen Eigenschaften hin untersucht. Hierzu wurden druckbeaufschlagte Fibrin-Patches mit nicht druckbeaufschlagen Fibrin-Patches untersucht, wobei die Druckbeaufschlagung 600 kPa [6 bar] für 20 Minuten betrug.

In der Abbildung 10 ist der Effekt der Verdichtung in Bezug auf Zugkraft, Dichte und Zugfestigkeit dargestellt. Es konnte gegenüber den nicht verdichteten Fibrin-Patches eine verbesserte Zugkraft und insbesondere Zugfestigkeit (nahezu Vervierfachung) erreicht werden. Weitere Untersuchungen ergaben, dass Patches mit steigender Fibrinkonzentration eine größere Zugkraft aufweisen.

Die untersuchten Fibrin-Patches wurden dabei gleichzeitig gegossen, ein Teil wurde ohne Druckbeaufschlagung, ein Teil mit Druckbeaufschlagung auspolimerisiert, wobei bei beiden Patches die Möglichkeit gegeben wurde, dass Flüssigkeit abfließen kann.

## Patentansprüche

1. Verfahren zur Herstellung eines bioartifiziellen und azellulären Konstrukts auf Fibrinbasis mit den Schritten:
- Bereitstellung einer Fibrinogen-haltigen und zellfreien Zusammensetzung;
- Bereitstellung einer Thrombin-haltigen und zellfreien Zusammensetzung;
- Aufbringen der Fibrinogen-haltigen zellfreien und der Thrombin-haltigen zellfreien Zusammensetzungen auf eine Oberfläche;
- Druckbeaufschlagung der auf der Oberfläche aufgebrachten Mischung der Zusammensetzungen zumindest solange, bis die Fibrinbildung im Wesentlichen abgeschlossen ist;
- Entfernen des azellulären bioartifiziellen Konstrukts auf Fibrinbasis von der Oberfläche, wobei die Beaufschlagung flächig auf die auf der Oberfläche aufgebrachten Zusammensetzungen erfolgt, wobei die Druckbeaufschlagung mit einem relativen Druck von mindestens 100 kPa [1 bar] erfolgt und wobei die aufgebrachte Schicht eine Dicke von mindestens 1 mm aufweist.

2. Verfahren zur Herstellung eines bioartifiziellen und azellulären Konstrukts auf Fibrinbasis nach Anspruch 1, wobei die Druckbeaufschlagung mit einem relativen Druck von mindestens 500 kPa [5 bar] erfolgt.

3. Verfahren nach einem der vorherigen Ansprüche, wobei die Zusammensetzungen, zusammen oder getrennt, mehrfach aufgebracht werden.

4. Verfahren nach Ansprüchen 1 bis 3, wobei als Zusammensetzungen, die die oberste Schicht ausbilden, zuerst eine Thrombin-haltige Zusammensetzung aufgebracht wird und anschließend eine Heparin-haltige Zusammensetzung.

5. Verfahren nach einem der vorherigen Ansprüche, wobei die Fibrinogen-haltige Zusammensetzung eine ist, wobei das Fibrinogen aus Plasma erhalten ist.

6. Verfahren nach einem der vorherigen Ansprüche, wobei die aufgebrachte Schicht eine Dicke von mindestens 1,5mm aufweist.

7. Verfahren nach einem der vorherigen Ansprüche, wobei mehrere Schichten nacheinander aufgebracht werden und wobei diese Schichten unterschiedliche Anteile an Thrombin-haltiger Zusammensetzung und Fibrinogenhaltiger Zusammensetzung aufweisen können und/oder wobei mindestens eine Fibrin-haltige Schicht aufgebracht wird und mindestens eine nicht-Fibrin-haltige Schicht, insbesondere andere biologische Materialien oder synthetische Materialen aufweisende Schicht, aufgebracht wird.

8. Verfahren nach einem der vorherigen Ansprüche, wobei die Fibrinogen-haltige Zusammensetzung eine Lösung ist, die Fibrinogen in einer Konzentration von maximal 60 mg/ml, wie maximal 20mg/ml aufweist.

9. Verfahren nach einem der vorherigen Ansprüche, wobei das Konstrukt ein rohrförmiges Konstrukt ist.

10. Verfahren nach einem der vorherigen Ansprüche, wobei eine Form mit der auf der Oberfläche aufgebrachten Zusammensetzung Bohrungen aufweist.

11. Verfahren nach Anspruch 10, wobei die Bohrungen mit einer semipermeablen Membran abgeschlossen sind.

12. Bioartifizielles, azelluläres Konstrukt auf Fibrinbasis erhältlich mit einem Verfahren nach einem der Ansprüche 1 bis 11, insbesondere wobei dieses als Patch oder als röhrenförmiger Hohlkörper ausgebildet ist.

13. Bioartifizielles, azelluläres Konstrukt auf Fibrinbasis nach Anspruch 12, wobei dieses eine Gefäßprothese ist.

14. Verwendung eines bioartifiziellen, azellulären Konstrukts erhältlich mit einem Verfahren der Ansprüche 1 bis 11 als Matrix für *in vitro* Herstellung einer Gefäßprothese.

15. Bioartifizielles, azelluläres Konstrukt zur Verwendung in einem Verfahren zur Implantation hiervon in einem Individuum umfassend den Schritt des Implantierens des Konstruktes nach einem der Ansprüche 12 oder 13 in das Individuum.

## Claims

1. Method for producing a fibrin-based bioartificial and acellular construct, comprising the following steps:
- provision of a fibrinogen-containing and cell-free composition;
- provision of a thrombin-containing and cell-free composition;
- application of the fibrinogen-containing cell-free and the thrombin-containing cell-free compositions to a surface;
- pressurization of the mixture of compositions applied to the surface at least until the fibrin formation is essentially completed; and
- removal of the fibrin-based acellular bioartificial construct from the surface, wherein pressure is applied to the surface and wherein the pressurization takes place at a relative pressure of at least 100 kPa (1 bar) and the applied layer has a thickness of at least 1 mm.

2. Method for producing a fibrin-based bioartificial and acellular construct, according to claim 1, wherein the pressurization takes place at a relative pressure of at least 500 kPa (5 bar).

3. Method according to one of the preceding claims, wherein the compositions, together or separately, are applied multiple times.

4. Method according to one of claims 1 through 3, wherein a thrombin-containing composition is first applied, followed by a heparin-containing composition, as compositions forming the uppermost layer.

5. Method according to one of the preceding claims, wherein the fibrinogen-containing composition is a composition in which the fibrinogen is obtained from plasma.

6. Method according to one of the preceding claims, wherein the applied layer has a thickness of at least 1.5 mm.

7. Method according to one of the preceding claims, wherein a plurality of layers is applied successively and wherein these layers can contain different proportions of the thrombin-containing composition and the fibrinogen-containing composition and/or wherein at least one fibrin-containing layer is applied and at least one non-fibrin-containing layer, more particularly a layer comprising other biological materials or synthetic materials, is applied.

8. Method according to one of the preceding claims, wherein the fibrinogen-containing composition is a solution that contains the fibrinogen in a concentration of at most 60 mg/ml, such as at most 20 mg/ml.

9. The method according to any one of the preceding claims, wherein the construct is a tubular construct.

10. The method according to any one of the preceding claims, wherein the mold with the composition applied to the surface comprises through holes.

11. The method according to claim 1 whereby the through holes are sealed with a semipermeable material.

12. Bioartificial, acellular fibrin-based construct obtainable by a method according to one of claims 1 through 11, in particular, wherein said construct is configured as a patch or a tubular hollow body.

13. Bioartificial, acellular fibrin-based construct according to claim 12, wherein said construct is a vascular graft.

14. Use of a bioartificial, acellular construct obtainable by a method according to claims 1 through 11 as a matrix for *in vitro* production of tissue replacement or a vascular graft.

15. Bioartificial acellular construct for use in a method of implanting the same in an individual comprising the step of implanting the construct of any one of claims 12 or 13 into the individual.

## Revendications

1. Procédé de fabrication d'une construction bioartificielle et acellulaire à base de fibrine, comprenant les étapes suivantes :
- fourniture d'une composition contenant du fibrinogène et exempte de cellules ;
- fourniture d'une composition contenant de la thrombine et exempte de cellules ;
- application des compositions exemptes de cellules contenant du fibrinogène et exemptes de cellules contenant de la thrombine sur une surface ;
- la pressurisation du mélange de compositions appliqué à la surface au moins jusqu'à ce que la formation de fibrine soit essentiellement terminée ; et
- le retrait de la construction bioartificielle acellulaire à base de fibrine de la surface, dans lequel la pression est appliquée à la surface et dans lequel la pressurisation a lieu à une pression relative d'au moins 100 kPa (1 bar) et la couche appliquée a une épaisseur d'au moins 1 mm.

2. Procédé de production d'une construction bioartificielle et acellulaire à base de fibrine, selon la revendication 1, dans lequel la pressurisation a lieu à une pression relative d'au moins 500 kPa (5 bars).

3. Procédé selon l'une des revendications précédentes, dans lequel les compositions, ensemble ou séparément, sont appliquées plusieurs fois.

4. Procédé selon l'une des revendications 1 à 3, dans lequel on applique d'abord une composition contenant de la thrombine, puis une composition contenant de l'héparine, en tant que compositions formant la couche supérieure.

5. Procédé selon l'une des revendications précédentes, dans lequel la composition contenant du fibrinogène est une composition dans laquelle le fibrinogène est obtenu à partir de plasma.

6. Procédé selon l'une des revendications précédentes, dans lequel la couche appliquée présente une épaisseur d'au moins 1,5 mm.

7. Procédé selon l'une des revendications précédentes, dans lequel plusieurs couches sont appliquées successivement et dans lequel ces couches peuvent contenir différentes proportions de la composition contenant de la thrombine et de la composition contenant du fibrinogène et/ou dans lequel au moins une couche contenant de la fibrine est appliquée et au moins une couche ne contenant pas de fibrine, plus particulièrement une couche comprenant d'autres matériaux biologiques ou des matériaux synthétiques, est appliquée.

8. Procédé selon l'une des revendications précédentes, dans lequel la composition contenant du fibrinogène est une solution qui contient le fibrinogène à une concentration d'au plus 60 mg/ml, par exemple d'au plus 20 mg/ml.

9. Procédé selon l'une quelconque des revendications précédentes, dans lequel la construction est une construction tubulaire.

10. Procédé selon l'une quelconque des revendications précédentes, dans lequel le moule avec la composition appliquée sur la surface comprend des trous traversants.

11. Procédé selon la revendication 1, les trous traversants étant obturés par un matériau semi-perméable.

12. Construction bioartificielle acellulaire à base de fibrine pouvant être obtenue par un procédé selon l'une des revendications 1 à 11, en particulier, dans laquelle ladite construction est configurée comme un patch ou un corps creux tubulaire.

13. Construction bioartificielle acellulaire à base de fibrine selon la revendication 12, dans laquelle ladite construction est un greffon vasculaire.

14. Utilisation d'une construction bioartificielle, acellulaire, pouvant être obtenue par un procédé selon les revendications 1 à 11, comme matrice pour la production in vitro d'un remplacement tissulaire ou d'un greffon vasculaire.

15. Construction bioartificielle acellulaire destinée à être utilisée dans un procédé d'implantation de celle-ci chez un individu, comprenant l'étape consistant à implanter la construction selon l'une des revendications 12 ou 13 dans l'individu.
